# EUROPÄISCHE PATENTSCHRIFT

(11) **EP 0 929 624 B1**
(45) Veröffentlichungstag und Bekanntmachung des Hinweises auf die Patenterteilung: **09.01.2002**
(21) Anmeldenummer: 97910339.7
(22) Anmeldetag: 24.09.1997
(51) Int. Cl.: C11D 1/52, A61K 7/50, B03D 1/00, B01F 17/00

(54) **TENSIDHALTIGE FORMULIERUNGEN**
SURFACTANT-CONTAINING FORMULATIONS
FORMULATIONS RENFERMANT DES AGENTS TENSIOACTIFS

(30) Priorität: 30.09.1996 DE 19640186
(43) Veröffentlichungstag der Anmeldung: 21.07.1999
(73) Patentinhaber: Clariant GmbH, 65929 Frankfurt am Main (DE)
(72) Erfinder: TUROWSKI, Angelika, D-65777 Kelkheim (DE); LÖFFLER, Matthias, D-65527 Niedernhausen (DE); SCHOLZ, Hans, Jürgen, D-63755 Alzenau (DE); SKRYPZAK, Werner, D-65719 Hofheim (DE); PAPENFUHS, Bernd, 63179 Obertshausen (DE)
(86) Internationale Anmeldenummer: EP9705227
(87) Internationale Veröffentlichungsnummer: WO9814542

(56) Entgegenhaltungen:
- DE-A- 19 512 299
- DE-A- 19 519 705
- DE-C- 4 238 211
- US-A- 4 021 539

## Beschreibung

Die Verwendung von Fettsäure-N-alkyl-polyhydroxyalkylamiden und insbesondere von Fettsäure-N-methylglucamid ist bereits aus DE-A-4 430 085; DE-A-4 326 950; DE-A-4 432 366; DE-A-4 424 823; WO 92/6153; WO 92/6156; WO 92/6157; WO 92/6158; WO 92/6159 und WO 92/6160 bekannt. Wesentliche Vorteile der Fettsäure-N-methylglucamide sind neben ihrer hohen Reinigungskraft ihre gute biologische Abbaubarkeit und ihre Herstellung aus nachwachsenden Rohstoffen. Bekannt ist auch die Verwendung dieser Substanzgruppe als Verdicker (EP-A-285 768).

Nachteilig für die Anwendung und Formulierbarkeit ist eine begrenzte Löslichkeit dieser Substanzen, insbesondere ab einer Kettenlänge größer als C16. Bei höheren Konzentrationen in Wasser sind sie aufgrund der hohen Viskosität nur schwer zu handhaben. Höhere Temperaturen zur Absenkung der Viskosität führen jedoch zu verstärkter Hydrolyse.

WO-A-9747591, die unter Article 54(3) EPü fällt, beschreibt Säure-Addukte von Fettsäurepolyhydroxyalkylamiden und Kompositionen, die diese Säure-Addukte und vorzugsweise zusätliche Tenside enthalten.

Aus DE-A-4 238 207 und DE-A-4 238 211 ist die Verwendung von Fettsäurepolyhydroxyalkylamiden als Tenside bekannt, die sich von den zuvor erwähnten Fettsäure-N-alkyl-polyhydroxyalkylamiden dadurch unterscheiden, daß die Alkylgruppe durch eine Dialkylaminogruppe substituiert ist. Darüberhinaus sind diese Zuckertenside quaterniert.

Es wurde nun gefunden, daß sich die in DE-A-4 238 207 und DE-A-4 238 211 beschriebenen Zuckertenside auch in ihrer nichtquaternierten Form ausgezeichnet als Tenside eignen. Dieser Befund ist überraschend, da der genannte Stand der Technik hierbei keine Anregungen zur Verwendung der nicht-quatemierten Verbindungen dieser Art als Tenside gibt.

Gegenstand der Erfindung sind tensidhaltige Formulierungen, die als Tensid ein N-(3-Dialkylamino)-propyl-N-polyhydroxyalkylcarbonsäureamid der Formel 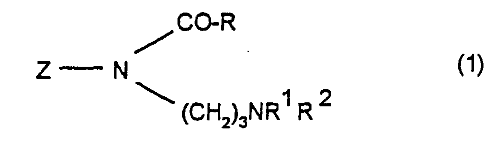 enthalten, worin R ein aliphatischer Rest mit 8 bis 24 C-Atomen ist, R¹ und R², die gleich oder verschieden sind, Alkyl mit 1 bis 4 C-Atomen oder Hydroxyalkyl mit 2 bis 4 C-Atomen und Z einen linearen Polyhydroxykohlenwasserstoffrest mit mindestens 3 OH-Gruppen, die auch alkoxyliert sein können, bedeuten sowie zusätlich enthaltend ein oder mehrere anionische, nicht-ionische, cationische und/oder amphotere Tenside.

Bevorzugt sind Verbindungen der Formel 1, in denen R¹ und R² Methyl und Z der Rest eines Zuckeralkohols ist, der sich von einem reduzierenden Mono- oder Disaccharid, insbesondere von der Glucose, ableitet.

Diese Verbindungen werden hergestellt, wie in DE-A-19 51 2249 angegeben, indem man ein Amin der Formel (2)

Z-NH-(CH₂)₃-NR¹R² (2)

unter Einführung des Restes R-CO acyliert, wobei R, R¹, R² und Z die obengenannten Bedeutungen haben.

Säureadditionsprodukte werden dann in einem zusätzlichen Schritt durch Zugabe einer geeigneten Säure erhalten. Beispiele für solche Säuren sind Mineralsäuren wie Salzsäure oder organische Säuren wie Carbonsäuren, (Essigsäure), Hydroxycarbonsäuren (Milchsäure), verzweigte, lineare, gesättigte oder ungesättigte Fettsäuren, Dicarbonsäuren (Maleinsäure, Bemsteinsäure, Adipinsäure), Polycarbonsäuren (Zitronensäure) oder Aminosäuren und deren Derivate.

Im Vergleich zu den nicht basisch substituierten bekannten Verbindungen haben diese Verbindungen den weiteren Vorteil, daß für die Abstimmung der hydrophilen Eigenschaften nicht nur der Rest Z, sondern auch der basisch substituierte Alkylenrest zur Verfügung steht und so die hydrophilen Eigenschaften im Vergleich zu den - durch den Rest R bewirkten - hydrophoben Eigenschaften noch besser maßgeschneidert werden können. Die Verbindungen der Formel 1 eignen sich ganz allgemein als Tenside, da sie ausgeprägte Tensideigenschaften, wie sehr gutes Schaumvermögen, gute Fettdispergierbarkeit (Schmutztitration) und gute Waschleistung bei gleichzeitig hoher Wasserhärtebeständigkeit und guter Hautverträglichkeit zeigen.

Sie eignen sich für alle Arten von tensidhaltigen Formulierungen, insbesondere für kosmetische Reinigungsmittelformulierungen und Haushaltsreiniger. Die erfindungsgemäßen Formulierungen enthalten die Verbindungen der Formel l vorzugsweise in einer Menge von 0,1 bis 99 Gew.-%, insbesondere von 1 bis 50 Gew.-%.

Bevorzugte erfindungsgemäße Formulierungen sind pulverförmige Universalwaschmittel (1 bis 30 Gew.-%), flüssige Universalwaschmittel (1 bis 70 Gew.-%), flüssige Feinwaschmittel (1 bis 50 Gew.-%), Avivagemittel (1 bis 50 Gew.-%), Handgeschirrspülmittel (1 bis 50 Gew.-%), Toilettenspüler (1 bis 50 Gew.-%), flüssige Reinigungs- und Desinfektionsmittel (1 bis 30 Gew.-%), Stückseifen zum Kombibat-Typ (1 bis 2 Gew.-%), Syndetseifen (1 bis 2 Gew.-%), Haarshampoos (1 bis 30 Gew.-%), Haarspülungen (1 bis 30 Gew.-%), Haarfärbemittel (1 bis 30 Gew.-%), Haarwellmittel (1 bis 30 Gew.-%), Schaumbäder (1 bis 30 Gew.-%), Gesichtsreinigungsmittel (1 bis 30 Gew.-%), Textil- und Faserhilfsmittel (1 bis 30 Gew.-%), Lederfettungsmittel (1 bis 30 Gew.-%), Flotationshilfsmittel (1 bis 30 Gew.-%) und Hilfsmittel für die Entwässerung von Feststoffen. Die Prozentangaben in den Klammern geben den bevorzugten Gehalt an Tensid der Formel 1 in diesen Formulierungen an.

Die erfindungsgemäßen Formulierungen enthalte eine Verbindung der Formel 1 kombiniert mit weiteren üblichen anionischen; nicht ionischen, kationischen und/oder amphoteren Tensiden. Das Mischungsverhältnis zwischen den Tensiden der Formel 1 und den übrigen Tensiden kann in breiten Grenzen schwanken, etwa im Gewichtsverhältnis von 1 zu 99 bis 99 zu 1, vorzugsweise von 80 zu 20 bis 20 zu 80. Die Gesamtkonzentration an Tensiden in den erfindungsgemäßen Formulierungen kann 1 bis 99, vorzugsweise 5 bis 50 Gew.-% betragen.

Als anionische Tenside kommen in Betracht Sulfonate, Sulfate, Carboxylate, Phosphate und Mischungen aus den genannten Verbindungen. Geeignete Kationen sind hierbei Alkalimetalle, wie z.B. Natrium oder Kalium oder Erdalkalimetalle, wie z.B. Calcium oder Magnesium sowie Ammonium, substituierte Ammoniumverbindungen, einschließlich Mono-, Di- oder Triethanolammoniumkationen und Mischungen der Kationen. Folgende Typen von anionischen Tensiden sind von besonderem Interesse:
Alkylestersulfonate, Alkylsulfate, Alkylethersulfate, Alkylbenzolsulfonate, sekundäre Alkansulfonate, Seifen wie im folgenden beschrieben.

Alkylestersulfonate sind unter anderem lineare Ester von C₈-C₂₀-Carboxylsäuren (d.h. Fettsäuren), welche mittels gasförmigem SO₃ sulfoniert werden, wie in "The Journal of the American Oil Chemists Society" 52 (1975), pp. 323-329 beschrieben wird. Geeignete Ausgangsmaterialien sind natürliche Fette wie z.B. Talg, Palmöl oder Cocosöl, können aber auch synthetischer Natur sein. Bevorzugte Alkylestersulfonate, speziell für Waschmittelanwendungen, sind Verbindungen der Formel 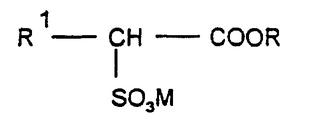 worin R¹ einen C₈-C₂₀-Kohlenwasserstoffrest, bevorzugt Alkyl und R einen C₁-C₆ Kohlenwasserstoffrest, bevorzugt Alkyl, darstellt. M steht für ein Kation, das ein wasserlösliches Salz mit dem Alkylestersulfonat bildet. Geeignete Kationen sind Natrium, Kalium, Lithium oder Ammoniumkationen, wie Monoethanolamin, Diethanolamin und Triethanolamin. Bevorzugt bedeuten R¹ C₁₀-C₁₆-Alkyl und R Methyl, Ethyl oder Isopropyl. Besonders bevorzugt sind Methylestersulfonate, in denen R¹ C₁₀-C₁₆-Alkyl bedeutet.

Alkylsulfate sind hier wasserlösliche Salze oder Säuren der Formel ROSO₃M, worin R bevorzugt ein C₁₀-C₂₄-Kohlenwasserstoffrest, bevorzugt C₁₀-C₂₀-Alkyl oder Hydroxyalkyl, besonders bevorzugt C₁₂-C₁₈ Alkyl- oder Hydroxyalkyl darstellt. M ist Wasserstoff oder ein Kation, z.B. ein Alkalimetallkation (z.B. Natrium, Kalium, Lithium), Ammonium oder substituiertes Ammonium z.B. Methyl-, Dimethyl- und Trimethylammoniumkationen und quatemäre Ammoniumkationen, wie Tetramethylammonium- und Dimethylpiperidiniumkationen und quatemäre Ammoniumkationen, abgeleitet von Alkylaminen wie Ethylamin, Diethylamin, Triethylamin und Mischungen davon. Alkylketten mit C₁₂-C₁₆ sind für niedrige Waschtemperaturen (z.B. unter ca. 50°C) und Alkylketten mit C₁₆-C₁₈ für höhere Waschtemperaturen (z.B. oberhalb ca. 50°C) bevorzugt.

Alkylethersulfate sind wasserlösliche Salze oder Säuren der Formel RO(A)ₘ SO₃M, worin R einen unsubstituierten C₁₀-C₂₄-Alkyl- oder Hydroxyalkylrest, bevorzugt einen C₁₂-C₂₀ Alkyl- oder Hydroxyalkylrest, besonders bevorzugt C₁₂-C₁₈-Alkyl- oder Hydroxyalkylrest darstellt. A ist eine Ethoxy- oder Propoxyeinheit, m ist eine Zahl größer als 0, vorzugsweise zwischen ca. 0,5 und ca. 6, besonders bevorzugt zwischen ca. 0,5 und ca. 3 und M ist ein Wasserstoffatom oder ein Kation wie z.B. Natrium, Kalium, Lithium, Calcium, Magnesium, Ammonium oder ein substituiertes Ammoniumkation. Spezifische Beispiele von substituierten Ammoniumkationen sind Methyl-, Dimethyl-, Trimethylammonium- und quatemäre Ammoniumkationen wie Tetramethylammonium und Dimethylpiperidiniumkationen, sowie solche, die von Alkylaminen, wie Ethylamin, Diethylamin, Triethylamin, Mischungen davon abgeleitet sind. Als Beispiele seien C₁₂-C₁₈-Fettalkoholethersulfate genannt, wobei der Gehalt an Ethylenoxid 1, 2, 2.5, 3 oder 4 mol pro mol Fettalkoholethersulfat beträgt, und in denen M Natrium oder Kalium ist.

In sekundären Alkansulfonaten kann die Alkylgruppe entweder gesättigt oder ungesättigt, verzweigt oder linear und gegebenenfalls mit einer Hydroxylgruppe substituiert sein. Die Sulfogruppe kann eine beliebige Position an der gesamten C-Kette einnehmen, wobei die primären Methylgruppen am Kettenanfang und Kettenende keine Sulfogruppen besitzen. Die bevorzugten sekundären Alkansulfonate enthalten lineare Alkylketten mit 9 bis 25 Kohlenstoffatomen, bevorzugt von 10 bis ca. 20 Kohlenstoffatome und besonders bevorzugt 13 bis 17 Kohlenstoffatome. Als Kation ist beispielsweise Natrium, Kalium, Ammonium, Mono-, Di- oder Triethanolammonium, Calcium oder Magnesium bevorzugt.

Weitere geeignete anionische Tenside sind Alkenyl- oder Alkylbenzolsulfonate. Die Alkenyl- oder Alkylgruppe kann verzweigt oder linear und gegebenenfalls mit einer Hydroxylgruppe substituiert sein. Die bevorzugten Alkylbenzolsulfonate enthalten lineare Alkylketten mit ca. 9 bis 25 Kohlenstoffatomen, bevorzugt von 10 bis 13 Kohlenstoffatome, das Kation ist Natrium, Kalium, Ammonium, Mono-, Di- oder Triethanolammonium, Calcium oder Magnesium und Mischungen davon. Für milde Tensidsysteme ist Magnesium als Kation bevorzugt, für Standardwaschanwendungen dagegen Natrium. Gleiches gilt für Alkenylbenzolsulfonate

Der Begriff anionische Tenside schließt auch Olefinsulfonate mit ein, die durch Sulfonierung von C₁₂-C₂₄-, vorzugsweise C₁₄-C₁₆-α-Olefinen mit Schwefeltrioxid und anschließende Neutralisation erhalten werden. Bedingt durch das Herstellverfahren, können diese Olefinsulfonate kleinere Mengen an Hydroxyalkansulfonaten und Alkandisulfonaten enthalten. Spezielle Mischungen von α-Olefinsulfonaten sind in US-3,332,880 beschrieben.

Weitere bevorzugte anionische Tenside sind Carboxylate, z.B. Fettsäureseifen und vergleichbare Tenside. Die Seifen können gesättigt oder ungesättigt sein und können verschiedene Substituenten, wie Hydroxylgruppen oder α-Sulfonatgruppen enthalten. Bevorzugt sind lineare gesättigte oder ungesättigte Kohlenwasserstoffreste als Anteil mit 6 bis 30, bevorzugt 10 bis 18 Kohlenstoffatomen.

Als anionische Tenside kommen weiterhin Salze von Acylaminocarbonsäuren in Frage, die durch Umsetzung von Fettsäurechloriden mit Natriumsarkosinat im alkalischen Medium entstehenden Acylsarcosinate; Fettsäure-Eiweiß-Kondensationsprodukte, die durch Umsetzung von Fettsäurechloriden mit Oligopeptiden erhalten werden; Salze von Alkylsulfamidocarbonsäuren, Salze von Alkyl- und Alkylarylethercarbonsäuren; C₈-C₂₄-Olefinsulfonate, sulfonierte Polycarboxylsäuren, hergestellt durch Sulfonierung der Pyrolyseprodukte von Erdalkalimetallcitraten, wie z.B. beschrieben in GB-1,082,179; Alkylglycerinsulfate, Fettacylglycerinsulfate, Alkylphenolethersulfate, primäre Paraffinsulfonate, Alkylphosphate, Alkyletherphosphate, Isethionate, wie Acylisethionate, N-Acyltauride, Alkylsuccinate, Sulfosuccinate, Monoester der Sulfosuccinate (besonders gesättigte und ungesättigte C₁₂-C₁₈-Monoester) und Diester der Sulfosuccinate (besonders gesättigte und ungesättigte C₁₂-C₁₈-Diester), Acylsarcosinate, Sulfate von Alkylpolysacchariden wie Sulfate von Alkylpolyglycosiden, verzweigte primäre Alkylsulfate und Alkylpolyethoxycarboxylate wie die der Formel RO(CH₂CH₂)ₖCH₂COO⁻M⁺, worin R C₈-C₂₂-Alkyl, k eine Zahl von 0 bis 10 und M ein Kation ist, Harzsäuren oder hydrierte Harzsäuren, wie Rosin oder hydriertes Rosin oder Tallölharze und Tallölharzsäuren. Weitere Beispiele sind in "Surface Active Agents and Detergents" (Vol. I und II, Schwartz, Perry und Berch) beschrieben.

Als nicht-ionische Tenside kommen beispielsweise folgende Typen in Frage:

Polyethylen-, Polypropylen- und Polybutylenoxidkondensate von Alkylphenolen.

Diese Verbindungen umfassen die Kondensationsprodukte von Alkylphenolen mit einer C₆-C₂₀-Alkylgruppe, die entweder linear oder verzweigt sein kann, mit Alkenoxiden. Bevorzugt sind Verbindungen mit ca. 5 bis 25 mol Ethylenoxid pro mol Alkylphenol. Kommerziell erhältliche Tenside diesen Typs sind z.B. Igepal® CO-630, Triton® X-45, X-114, X-100 und X102, und die ® Arkopal-N-Marken der Hoechst AG.

Kondensationsprodukte von aliphatischen Alkoholen mit ca. 1 bis ca. 25 mol Ethylenoxid.

Die Alkylkette der aliphatischen Alkohole kann linear oder verzweigt, primär oder sekundär sein, und enthält im allgemeinen ca. 8 bis ca. 22 Kohlenstoffatome. Besonders bevorzugt sind die Kondensationsprodukte von C₁₀-C₂₀-Alkoholen, mit ca. 2 bis ca. 18 mol Ethylenoxid pro mol Alkohol. Die Alkylkette kann gesättigt oder auch ungesättigt sein. Die Alkoholethoxilate können eine enge ("Narrow Range Ethoxylates") oder eine breite Homologenverteilung des Ethylenoxides ("Broad Range Ethoxylates") aufweisen. Beispiele von kommerziell erhältlichen nichtionischen Tensiden dieses Types sind Teritol® 15-S-9 (Kondensationsprodukt eines C₁₁-C₁₅ linearen sekundären Alkohols mit 9 mol Ethylenoxid), Tergitol® 24-L-NMW (Kondensationsprodukt eines C₁₂-C₁₄-linearen primären Alkohols mit 6 mol Ethylenoxid mit enger Molgewichtsverteilung). Ebenfalls unter diese Produktklasse fallen die Genapol® -Marken der Hoechst AG.

Kondensationsprodukte von Ethylenoxid mit einer hydrophoben Basis, gebildet durch Kondensation von Propylenoxid mit Propylenglykol.

Der hydrophobe Teil dieser Verbindungen weist bevorzugt ein Molekulargewicht zwischen 1500 und 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhätliche Beispiele dieser Produktklasse sind die Pluronic® -Marken der BASF und die ® Genapol PF-Marken der Hoechst AG.

Kondensationsprodukt von Ethylenoxid mit einem Reaktionsprodukt von Propylenoxid und Ethylendiamin.

Die hydrophobe Einheit dieser Verbindungen besteht aus dem Reaktionsprodukt von Ethylendiamin mit überschüssigem Propylenoxid und weist im allgemeinen ein Molekulargewicht von 2500 bis 3000 auf. An diese hydrophobe Einheit wird Ethylenoxid addiert, bis das Produkt einen Gehalt von ca. 40 bis ca. 80 Gew.-% Polyoxyethylen und ein Molekulargewicht von ca. 5000 bis ca. 11000 aufweist. Kommerziell erhältliche Beispiele dieser Verbindungsklase sind die ® Tetronic-Marken der BASF und die ® Genapol PN-Marken der Hoechst AG.

### Semipolare nichtionische Tenside

Diese spezielle Kategorie von nichtionischen Verbindungen umfaßt wasserlösliche Aminoxide, wasserlösliche Phosphinoxide und wasserlösliche Sulfoxide jeweils mit einem Alkylrest von 10 bis 18 Kohlenstoffatomen. Semipolare nichtionische Tenside sind auch Aminoxide der Formel 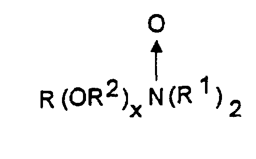 R ist hierbei eine Alkyl-Hydroxyalkyl- oder Alkylphenolgruppe mit jeweils 8 bis 22 Kohlenstoffatomen, R² ist eine Alkylen- oder Hydroxyalkylengruppe mit ca. 2 bis 3 Kohlenstoffatomen oder Mischungen hiervon, jeder Rest R¹ ist eine Alkyl- oder Hydroxyalkylgruppe mit 1 bis 3 Kohlenstoffatomen oder eine Polyethylenoxidgruppe mit 1 bis 3 Ethylenoxideinheiten. Die R¹-Gruppen können miteinander über ein Sauerstoff- oder Stickstoffatom verbunden sein und somit einen Ring bilden. Aminoxide dieser Art sind besonders C₁₀-C₁₈-Alkyldimethylaminoxide und C₈-C₁₂-Alkoxiethyl-Dihydroxyethylaminoxide.

### Fettsäureamide

Fettsäureamide besitzen die Formel 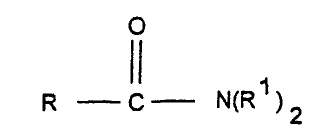 worin R eine Alkylgruppe mit 7 bis 21, bevorzugt 9 bis 17 Kohlenstoffatomen ist und jeder Rest R¹ Wasserstoff, C₁-C₄-Alkyl, C₁-C₄-Hydroxyalkyl oder (C₂H₄O)ₓH bedeutet, wobei x von 1 bis 3 variiert. Bevorzugt sind C₈-C₂₀-Amide, -monoethanolamide, -diethanolamide und-isopropanolamide.

Weitere geeignete nicht-ionische Tenside sind insbesondere Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, alkoxylierte Triglycamide, Mischether oder Mischformyle, Fettsäure-N-alkylglucamide, Proteinhydrolysate, Phosphinoxide oder Dialkylsulfoxide.

Typische Beispiele für amphotere bzw. zwitterionische Tenside sind Alkylbetaine, Alkylamidbetaine, Aminopropionate, Aminoglycinate, oder amphotere Imidiazolinium-Verbindungen der Formel 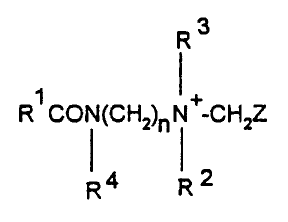 worin R¹ C₈-C₂₂-Alkyl- oder -Alkenyl, R² Wasserstoff oder CH₂CO₂M, R³ CH₂CH₂OH oder CH₂CH₂OCH₂CH₂COOM, R⁴ Wasserstoff, CH₂CH₂OH oder CH₂CH₂COOM, Z CO₂M oder CH₂CO₂M, n 2 oder 3, bevorzugt 2, M Wasserstoff oder ein Kation wie Alkalimetall, Erdalkalimetall, Ammoniak oder Alkanolammonium bedeutet.

Bevorzugte amphotere Tenside dieser Formel sind Monocarboxylate und Dicarboxylate. Beispiele hierfür sind Cocoamphocarboxypropionat, Cocoamidocarboxypropionsäure, Cocoamphocarboxyglycinat (auch als Cocoamphodiacetat bezeichnet) und Cocoamphoacetat.

Weitere bevorzugte amphotere Tenside sind Alkyldimethylbetaine und Alkyldipolyethoxybetaine mit einem Alkylrest, der linear oder verzweigt sein kann, mit 8 bis 22 Kohlenstoffatomen, bevorzugt mit 8 bis 18 Kohlenstoffatomen und besonders bevorzugt mit 12 bis 18 Kohlenstoffatomen. Diese Verbindungen werden z.B. von der Hoechst AG unter dem Handelnamen ® Genagen LAB vermarktet.

Geeignete kationische Tenside sind substituierte oder unsubstituierte geradkettige oder verzweigte quartäre Ammoniumsalze vom Typ R¹N(CH₃)₃^{⊕}X^{⊖}, R¹R²N(CH₃)₂^{⊕}X^{⊖}, R¹R²R³N(CH₃)^{⊕}X^{⊖} oder R¹R²R³R⁴N^{⊕}X^{⊖}. Die Reste R¹' R²' R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit 1 bis 4 C-Atomen, Phenyl, C₂- bis C₁₈-Alkenyl, C₇- bis C₂₄-Aralkyl, (C₂H₄O)ₓH, wobei x von 1 bis 3 bedeutet, ein oder mehrere Estergruppen enthaltende Alkylreste oder cyclische quartäre Ammoniumsalze sein. X ist ein geeignetes Anion.

Die erfindungsgemäßen Formulierungen enthalten, je nach Anwendungszweck, neben den genannten Tensiden noch die jeweils spezifischen Hilfs- und Zusatzstoffe. So enthalten Wasch- und Reinigungsformulierungen beispielsweise Builder, Salze, Bleichmittel, Bleichaktivatoren, optische Aufheller, Vergrauungsinhibitoren, Lösungsvermittler und Enzyme.

Übliche Builder sind Natriumaluminiumsilikate (Zeolithe), Schichtsilikate, Phosphate, Phosphonate, Ethylendiamintetraessigsäure, Nitrilotriacetat, Citronensäure und/oder Polycarboxylate.

Als Salze bzw. Stellmittel kommen beispielsweise Natriumsulfat, Natriumcarbonat oder Natriumsilikat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

In kosmetischen oder pharmazeutischen Formulierungen können neben den genannten Tensiden unter anderem Verdicker, Feuchtigkeitsmittel, biogene Wirkstoffe, Filmbildner, Konditioniermittel, Perlglanzmittel, Konservierungsmittel, Parfüm oder Farbstoffe enthalten sein.

Haarshampoos, Haarlotionen oder Dusch- und Schaumbäder können als weitere Hilfs- und Zusatzstoffe Emulgatoren wie etwa alkoxylierte Fettalkohole oder Sorbitanester enthalten.

Als Überfettungsmittel können Substanzen wie beispielsweise polyoxethylierte Lanolinderivate, Lecithinderivate und Fettsäurealkanolamide verwendet werden, wobei die letzteren gleichzeitg als Schaumstabilisatoren dienen.

Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und dieester von Fettsäuren, Polyacrylate, Polyvinylalkohol und Polyvinylpyrrolidon sowie Elektrolyte wie Kochsalz und Ammoniumchlorid.

Unter biogenen Wirkstoffen sind beispielsweise Pflanzenextrakte und Vitaminkomplexe zu verstehen.

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate und ähnliche Verbindungen.

Eine weitere Komponente, die in den Formulierungen, enthalten sein kann, sind nicht flüchtige, flüssige Silicone. Dieses kann entweder ein Polyalkylsiloxan, ein Polyarylsiloxan, ein Polyalkylarylsiloxan oder ein Polyethersiloxan-Copolymer sein und wird in einer Menge von 0,1 % bis 10,0 %, bevorzugt in einer Menge von 0,5 % bis 5,0 % eingesetzt. Mischungen dieser Flüssigkeiten können ebenso verwendet werden und sind auch für bestimmte Anwendungen von Vorteil. Die dispergierten Siliconteilchen sollten unlöslich in der Shampoomatrix sein. Die wichtigsten nicht flüchtigen Polyalkylsiloxane, die eingesetzt werden können, sind z.B. Polydimethylsiloxane mit Viskositäten von 5 bis 600.000 Centistokes, vorzugsweise von 350 und 100.000 Centistokes, bei 25°C. Ein geeignetes, im wesentlichen nicht flüchtiges Polyethersiloxan, ist z.B. ein mit Polypropylenoxid modifiziertes Dimethylpolysiloxan. Es können auch Addukte mit Ethylenoxid und/oder Propylenoxid eingesetzt werden.

Geeignete Silicone werden beispielsweise in US-2,826,551, US-3,946,500, US-4,364,837 und in GB-849,433 beschrieben.

Weiterhin kann Silicon-Gum erfindungsgemäß eingesetzt werden. Silicon-Gums sind in US-4,152,416 beschrieben. Geeignete Silicon-Gums werden ebenso beschrieben in den Produktdatenblättem SE 30, SE 33, SE 54 und SE 76 der Firma General Electric. "Silicon Gum" bedeutet hochmolekulare Polydiorganosiloxane mit einer Molmasse von ca. 200.000 bis 1.000.000. Spezielle Beispiele sind Polydimethylsiloxan, (Polydimethylsiloxan)(Methylvinylsiloxan)-Copolymer, Poly(dimethylsiloxan)(Diphenyl)(Methylvinylsiloxan)-Copolymer und ihre Mischungen, Mischungen von Siliconflüssigkeiten und Silicon-Gums sind ebenfalls geeignet.

Als Konservierungsmittel eignen sich beispielsweise Phenoxyethanol, Formaldehydlösung, Parabene, Pentadiol oder Sorbinsäure.

Als Perlglanzmittel kommen beispielsweise Glycoldistearinsäureester wie Ethylenglycoldistearat, aber auch Fettsäuremonoglycolester in Betracht.

Als Farbstoffe können die für kosmetischen Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung eingesetzt.

Der Gesamtanteil der Hilfs- und Zusatzstoffe kann 1 bis 50, vorzugsweise 5 bis 40 Gew.-%, bezogen auf die tensidhaltige Formulierung betragen.

### Beispiele

Im folgenden werden zunächst einige Richtrezepturen für eine Reihe von speziellen Formulierungen gemäß der Erfindung angegeben. Daran anschließend folgen einige definierte Formulierungen. In allen Beispielen bedeutet DMAP-GA ein Zuckertensid der Formel 1 wie zuvor angegeben worin Z ein Glucoserest, R ein C₁₂/C₁₄-Fettsäurealkylrest, wenn nicht anders angegeben und R¹ und R² jeweils Methyl bedeuten. Alle Prozentangaben sind Gewichtsprozente.

| Rasiercreme 1: | | |
|---|---|---|
| Zusammensetzung | Stearinsäure | 20 bis 40 % |
| | Cocosfettsäure | 6 bis 10 % |
| | DMAP-GA | 1 bis 45 % |
| | Glycerin | 5 bis 15 % |
| | Kaliumhydroxid | 2 bis 6 % |
| | Natriumhydroxid | 1 bis 3 %. |
| | Pflanzen- oder | |
| | Mineralöl | 1 bis 5 % |
| | Wasser | ad 100 % |

| Rasiercreme 2: | | |
|---|---|---|
| Zusammensetzung | Glycerin-monostearat | 10 bis 35 % |
| | DMAP-GA | 1 bis 45 % |
| | Mineralöl | 5 bis 15 % |
| | Glycerin | 1 bis 10 % |
| | Wasser | ad 100 % |

| Rasierlotion: | | |
|---|---|---|
| Zusammensetzung | Cellulosealkylether | 70 bis 75 % |
| | DMAP-GA | 1 bis 5 % |
| | Mineralöl | 10 bis 20 % |
| | Glycerin | 3 bis 10 % |
| | Wasser | ad 100 % |

| Duschgel: | | |
|---|---|---|
| Zusammensetzung | Cellulosealkylether | 5 bis 10 % |
| | Na-Laurylethersulfat | 2 bis 5 % |
| | DMAP-GA | 1 bis 45 % |
| | Cocoylamidopropylbetain | 8 bis 15 % |
| | Ethylenglykoldistearat | 4 bis 10 % |
| | Isopropylpalmitat | 0,5 bis1 % |
| | Feuchtmittel | 0,25 bis 0,5 % |
| | Konservierungsmittel | 0,05 bis 0,1 % |
| | Natriumchlorid | 3 bis 5 % |
| | Wasser | ad 100 % |

| Klarer Allzweckreiniger: | | |
|---|---|---|
| Zusammensetzung | DMAP-GA | 0,1 bis 15 % |
| | Anionische Tenside | 0 bis 25 % |
| | Amphotere Tenside | 0 bis 5 % |
| | Nichtionische Tenside | 0,5 bis 15 % |
| | Parfümöl | 0 bis 1 % |
| | Konservierungsmittel | 0 bis 1 % |
| | Wasser ad | 100 % |

| Handgeschirrspülmittel: | | |
|---|---|---|
| Zusammensetzung | DMAP-GA | 0,1 bis 15 % |
| | Anionische Tenside | 0 bis 40 % |
| | Amphotere Tenside | 0 bis 15 % |
| | Nichtionische Tenside | 0 bis 15 % |
| | Aminoxide | 0 bis 15 % |
| | Parfümöl | 0 bis 1 % |
| | Konservierungsmittel | 0 bis 1 % |
| | Natriumchlorid | 0 bis 5 % |
| | Wasser ad | 100 % |

| Flüssigwaschmittel: | | |
|---|---|---|
| Zusammensetzung | DMAP-GA | 0,1 bis 15 % |
| | Anionische Tenside | 0 bis 40 % |
| | Nichtionische Tenside | 0 bis 40 % |
| | Stellmittel | 0 bis 15 % |
| | Enzyme | 0 bis 15 % |
| | Parfümöl | 0 bis 1 % |
| | Konservierungsmittel | 0 bis 1 % |
| | Farbstoff | 0 bis 1 % |
| | Wasser ad | 100 % |

### Beispiel 1: Klares Duschgel mit 15 % Wirkstoffgehalt (WAS):

| Zusammensetzung: | | |
|---|---|---|
| A | DMAP-GA | 4.00 % |
| B | Wasser | 48.85 % |
| C | ® Genapol LRO flüssig | 40.35 % |
| | PEG 400 | 5.00 % |
| | Parfümöl | 0.30 % |
| | Konservierungsmittel | q.s. |
| | Farbstofflösung | q.s. |
| D | Citronensäure | q.s. |
| E | Natriumchlorid | 1.50 % |

| Herstellung: | |
|---|---|
| I | A unter Erwärmung in B auflösen |
| II | Die Komponenten von C nacheinander in I einrühren |
| III | Den pH-Wert mit D regulieren, anschließend die Viskosität mit E einstellen |

### Beispiel 2: Klares Duschgel mit 16 % Wirkstoffgehalt (WAS) ohne Natriumchloridzusatz

| Zusammensetzung: | | |
|---|---|---|
| A | DMAP-GA | 6.00 % |
| B | Wasser | 54.50 % |
| C | ® Genapol LRO flüssig | 30.00 % |
| | ® Genagen CAB 818 | 5.00 % |
| | ® Hostapon KCG | 4.00 % |
| | Parfümöl | 0.50 % |
| | Konservierungsmittel | q.s. |
| | Farbstofflösung | q.s. |
| D | Citronensäure | q.s. |

### Herstellung: analog Beispiel 1

### Beispiel 3: Duschbad mit Perlglanz und 16 % Wirkstoffgehalt (WAS) ohne Zusatz von Natriumchlorid

| Zusammensetzung: | | |
|---|---|---|
| A | DMAP-GA | 6.00 % |
| B | Wasser | 54.70 % |
| C | ® Genapol LRO flüssig | 35.00 % |
| | ® Genapol TSM | 4.00 % |
| | Parfümöl | 0.30 |
| | Konservierungsmittel | q.s. |
| | Farbstofflösung | q.s. |
| D | Citronensäure | q.s. |

### Herstellung analog Beispiel 1

### Beispiel 4: Klares Antischuppenshampoo mit 12.5 % Wirkstoffgehalt (WAS) ohne Natriumchlorid

| Zusammensetzung: | | |
|---|---|---|
| A | DMAP-GA | 5.00 % |
| | ® Octopirox | 0.50 % |
| B | Wasser | 64.20 % |
| C | ® Genapol LRO flüssig | 30.00 % |
| | Parfümöl | 0.30 % |
| | Konservierungsmittel | q.s. |
| | Farbstofflösung | q.s. |
| D | Citronensäure | |

### Herstellung analog Beispiel 1

### Beispiel 5: Klares Antischuppenshampoo mit 12.5 % Wirkstoffgehalt (WAS):

| Zusammensetzung: | | |
|---|---|---|
| A | DMAP-GA | 2.50 % |
| | ® Octopirox | 0.50 % |
| B | Wasser | 57.10 % |
| C | ® Genapol LRO flüssig | 30.00 % |
| | ® Medialan LD | 6.60 % |
| | Parfümöl | 0.30 % |
| | Konservierungsmittel | q.s. |
| | Farbstofflösung | q.s. |
| D | Citronensäure | q.s. |
| E | Natriumchlorid | 3.00 % |

### Herstellung analog Beispiel 1

### Beispiel 6: Klares Antischuppenshampoo mit 12.5 % Wirkstoffgehalt (WAS):

| Zusammensetzung: | | |
|---|---|---|
| A | DMAP-GA | 2.50 % |
| | ® Octopirox | 0.50 % |
| B | Wasser | 61.35 % |
| C | ® Genapol LRO flüssig | 30.00 % |
| | ® Genapol SBE | 3.35 % |
| | Parfümöl | 0.30 % |
| | Konservierungsmittel | q.s. |
| | Farbstofflösung | q.s. |
| D | Citronensäure | q.s. |
| E | Natriumchlorid | 2.00 % |

### Herstellung analog Beispiel 1

### Beispiel 7: Klares Haarshampoo mit 15 % Wirkstoffgehalt (WAS):

| Zusammensetzung: | | |
|---|---|---|
| A | DMAP-GA | 5.00 % |
| B | Wasser | 53.70 % |
| C | ® Genapol LRO flüssig | 35.00 % |
| | ® Genapol SBE | 5.00 % |
| | Parfümöl | 0.30 % |
| | Konservierungsmittel | q.s. |
| | Farbstofflösung | q.s. |
| D | Citronensäure | q.s. |
| E | Natriumchlorid | 1.00 % |

### Herstellung analog Beispiel 1

### Beispiel 8: Klarer Allzweckreiniger mit 10 % Wirkstoffgehalt (WAS)

| Zusammensetzung: | | |
|---|---|---|
| A | DMAP-GA | 1.50 % |
| B | Wasser | 90.70 % |
| C | ® LRO flüssig | 1.50 % |
| | ® Hostapur SAS 60 | 6.00 % |
| | Parfümöl | 0.30 % |
| | Konservierungsmittel | q.s. |
| | Farbstofflösung | q.s. |
| D | Citronensäure | q.s. |

### Herstellung analog Beispiel 1

### Beispiel 9: Klares Geschirrspülmittel mit 20 % Wirkstoffgehalt (WAS)

| Zusammensetzung: | | |
|---|---|---|
| A | DMAP-GA | 2.00% |
| B | Wasser | 62.70 % |
| C | ® Genapol LRO flüssig | 8.00 % |
| | ® Hostapur SAS 60 | 25.00 % |
| | Parfümöl | 0.30 % |
| | Konservierungsmittel | q.s. |
| | Farbstofflösung | q.s. |
| D | Citronensäure | q.s. |
| E | Natriumchlorid | 2.00 % |

### Herstellung analog Beispiel 1

### Beispiel 10: Klares Geschirrspülmittel mit 32.5 % Wirkstoffgehalt (WAS) ohne Chloridzusatz

| Zusammensetzung | | |
|---|---|---|
| A | DMAP-GA | 5.00 % |
| B | Wasser | 53.70 % |
| C | ® Genapol LRO flüssig | 6.00 % |
| | ® Hostapur SAS 60 | 35.00 % |
| | Parfümöl | 0.30 % |
| | Konservierungsmittel | q.s. |
| | Farbstofflösung | q.s. |
| D | Citronensäure | q.s. |

### Herstellung analog Beispiel 1

### Beispiel 11 (Vergleich): Duschbad mit Perlglanz, 14 % WAS

| Zusammensetzung: | | |
|---|---|---|
| A | ® GENAPOL LRO flüssig (Hoechst AG) | 35.00 % |
| B | C12/14-DMAP-GA x Milchsäure (Hoechst AG) | 7.60 % |
| | ® GENAPOL PGL (Hoechst AG) | 4.00 % |
| | Parfümöl | 0.30 % |
| | Wasser | 53.10% |
| | Konservierungsmittel | q.s. |
| | Farbstofflösung | q.s. |
| C | Milchsäure | q.s. |

| Herstellung | |
|---|---|
| I | Die Komponenten von B nacheinander in A einrühren. |
| II | Den pH-Wert mit C regulieren. |

### Beispiel 12 (Vergleich): Duschgel klar, 14 % WAS

| Zusammensetzung: | | |
|---|---|---|
| A | ® GENAPOL LRO flüssig (Hoechst AG) | 30.00 % |
| B | C12/14-DMAP-GAx Salzsäure (Hoechst AG) | 8.4 % |
| | ® GENAGEN CAB 818 (Hoechst AG) | 5.00 % . |
| | Parfümöl | 0.30 % |
| | Wasser | 56.30 % |
| | Konservierungsmittel | q.s. |
| | Farbstofflösung | q.s. |
| C | Salzsäure | q.s. |

| Herstellung | |
|---|---|
| I | Die Komponenten von B nacheinander in A einrühren. |
| II. | Den pH-Wert mit C regulieren. |

### Beispiel 13 (Vergleich): Antischuppenshampoo klar, 14 % WAS

| Zusammensetzung: | | |
|---|---|---|
| A | ® OCTOPIROX (Hoechst AG) | 0.50 % |
| B | ® GENAPOL LRO flüssig (Hoechst AG) | 35.00 % |
| C | C12/14-DMAP-GA x Salzsäure (Hoechst AG) | 8.40 % |
| | Parfümöl | 0.30 % |
| | Wasser | 55.80 % |
| | Konservierungsmittel | q.s. |
| | Farbstofflösung | q.s. |
| D | Salzsäure | q.s. |

| Herstellung | |
|---|---|
| I | A in B lösen. |
| II | Die Komponenten von C nacheinander in I einrühren. |
| III | Den pH-Wert mit D regulieren. |

### Beispiel 14 (Vergleich): Haarshampoo klar, 14 % WAS

| Zusammensetzung: | | |
|---|---|---|
| A | ® GENAPOL LRO flüssig (Hoechst AG) | 35.00 % |
| B | C12/14-DMAP-GA x Milchsäure (Hoechst AG) | 7.60 % |
| | Parfümöl | 0.30 % |
| | Wasser | 57.10% |
| | Konservierungsmittel | q.s. |
| | Farbstofflösung | q.s. |
| C | Milchsäure | q.s. |

| Herstellung | |
|---|---|
| I | Die Komponenten von B nacheinander in A einrühren. |
| II | Den pH-Wert mit C regulieren |

### Beispiel 15: Vollwaschmittel

| Zusammensetzung: | |
|---|---|
| Alkylsulfat | 12 % |
| Seife | 1 % |
| Fettalkoholoxethylat | 4 % |
| DMAP-GA | 3 % |
| Natriumcarbonat | 6 % |
| Schichtsilikat SKS-6 | 14 % |
| Zeotith | 14 % |
| Natriumcitrat | 5 % |
| Natriumsulfat | 2 % |
| Natriumpercarbonat | 20 % |
| Bleichaktivator | 4 % |
| Polyacrylat (CP-5) | 6 % |
| Enzyme | 1 % |
| Wasser | ad 100 % |

### Beispiel 16: Feinwaschmittel

| Zusammensetzung | |
|---|---|
| Alkylbenzolsulfonat | 14 % |
| Alkylsulfat | 8 % |
| Seife | 2 % |
| Fettalkoholethoxylat | 4 % |
| DMAP-GA | 2 % |
| Natriumcarbonat | 1 % |
| Schichtsilikat SKS-6 | 5 % |
| Zeolith | 40 % |
| Natriumsulfat | 14 % |
| Enzyme | 1 % |
| Wasser | ad 100 % |

| Verzeichnis der eingesetzten Handelsprodukte: | |
|---|---|
| ® Genapol LRO flüssig | C₁₂/C₁₈-Alkyldiglycolethersulfat-Natriumsalz (ca. 27 % WAS) |
| ® Hostapur SAS 60 | Sekundäres Alkansulfonat-Natriumsalz (ca. 60 % WAS) |
| ® Genapol SBE | C₁₂/C₁₈-Alkylpolyglycolethersulfosuccinat-Dinatriumsalz (ca. 40 % WAS) |
| ® Medialan LD | Fettsäuresarcosid-Natriumsalz (ca. 30 % WAS) |
| ® Genapol TSM | Alkylethersulfat und Seidenglanzbilder |
| ® Genapol OA 080 | C12/C14-Fettalkoholethoxylat mit 8 EO |
| ® Genagen CAB 818 | Alkylamidopropylbetain (ca. 30 % WAS) |
| ® Hostapon KCG | N-Cocoylglutaminsäure-mono-Natriumsalz (ca. 25 % WAS) |
| PEG 400 | Polyethylenglycol (Molmasse ca. 400) |
| ® Octopirox | 1-Hydroxy-4-methyl-6-(2,4,4-trimethylpentyl)-2(1H)-pyridon, 2-Aminoethanolsalz (Antischuppenmittel) |

### Anwendungstechnische Untersuchungen

Es wurden das Schaumvermögen, die Calcium-Verträglichkeit und das Netzvermögen verschiedener Tenside einschließlich DMAP-GA geprüft.

### 1. Schaumvermögen

Das Schaumvermögen folgender Tenside:
DMAP-GA,
APG (C₁₂/C₁₄-Alkylpolyglykosid),
CAPB (C₈/C₁₈-Cocosalkylamidopropyl-betain)
wurde nach der Methode von Ross-Miles bei 37°C in Wasser mit unterschiedlicher Calcium-Härte geprüft. Die Konzentration der Tenside im Wasser betrug jeweils 0,3 g/L. In einem zusätzlichen Test wurde jeweils 1 ml Fett (Crisco + Olivenöl, 1:1) zugegeben. Es wurden folgende Werte (Schaumhöhe in mm) gemessen:

| | | DMAP-GA | APG | CAPB |
|---|---|---|---|---|
| 1 | 0° dH | 140 | 50 | 180 |
| 2 | 0° dH | 90 | 50 | 70 |
| 3 | 3° dH | 140 | 20 | 180 |
| 4 | 3° dH | 90 | 20 | 80 |
| 5 | 15° dH | 150 | 20 | 180 |
| 6 | 15° dH | 110 | 15 | 80 |
| 7 | 25° dH | 150 | 20 | 180 |
| 8 | 25° dH | 80 | 15 | 80 |

Die Werte in den Zeilen 1, 3, 5, 7 geben die Schaumhöhe ohne Fett, die Werte in den Zeilen 2, 4, 6, 8 die Schaumhöhe unter Zusatz von Fett an.

Nach der gleichen Methode wurden DMAP-GA-haltige Gemische der folgenden Zusammensetzung geprüft:
I. 1 Teil DMAP-GA + 4 Teile einer Mischung aus 7 Teilen C₁₂/C₁₄-Laurylethersulfat + 2 EO und 3 Teilen C₈/C₁₈-Cocosalkylamidopropylbetain;
II. 1 Teil C₁₂/C₁₄-Alkylpolyglykosid + 4 Teile einer Mischung aus 7 Teilen C₁₂/C₁₄-Laurylethersulfat + 2 EO und 3 Teilen C₈/C₁₈-Cocosalkylamidopropylbetain.

| | | I | II |
|---|---|---|---|
| 1 | 0° dH | 160 | 175 |
| 2 | 0° dH | 75 | 40 |
| 3 | 3° dH | 170 | 180 |
| 4 | 3° dH | 170 | 145 |
| 5 | 15° dH | 160 | 190 |
| 6 | 15° dH | 145 | 80 |
| 7 | 25° dH | 150 | 190 |
| 8 | 25° dH | 130 | 110 |

Die Werte in den Zeilen 1, 3, 5, 7 geben die Schaumhöhe ohne Fett, die Werte in den Zeilen 2, 4, 6, 8 unter Zusatz von Fett an.

Weiterhin wurde das Schaumvermögen nach Ross-Miles der folgenden Säureadditionsprodukte gemessen:
a) C₁₂/C₁₄-DMAP-GA-Milchsäureaddukt
b) C₁₂/C₁₄-DMAP-GA-HCl Addukt
c) C₁₆/C₁₈-DMAP-GA-Milchsäureaddukt
d) C₁₆/C₁₈-DMAP-GA-HCI Addukt

| | Schaumhöhe in mm | | | |
|---|---|---|---|---|
| | a) | b) | c) | d) |
| 1,0 | 250/250 | 245/245 | 185/180 | 190/190 |
| 0,1 | 195/195 | 200/195 | 160/160 | 150/145 |
| 0,03 | 35/10 | 45/15 | 65/65 | 80/75 |
| 0,006 | 15/5 | 10/5 | 15/10 | 25/25 |
| 0,002 | 5/0 | 5/0 | 15/10 | 15/10 |

| Schaumvolumen in ml | | | | |
|---|---|---|---|---|
| 1,0 | 450/440 | 520/510 | 80/70 | 130/120 |

Prüfbedingungen: Temperatur 37°C, Wasserhärte 15°dH; pH = 7

Die jeweils erste Zahl gibt den Anfangsmeßwert, die jeweils zweite Zahl den Meßwert nach 5 Minuten an.

Die Prüfergebnisse zeigen, daß auch die Säureaddukte ein sehr gutes Schaumverhalten zeigen.

Alle Zahlenangaben geben die Schaumhöhe in mm an.

Die Ausprüfungen nach Ross-Miles zeigen, daß sowohl DMAP-GA als freies Amidamin und auch als Säureaddukt allein sowie Mischungen dieser Tenside mit Cotensiden selbst unter Fettbelastung ein sehr gutes Schaumvermögen besitzen.

### 2. Calcium-Verträglichkeit

Die Bestimmung der Beständigkeit der Tenside gegenüber hartem Wasser erfolgte, indem Lösungen mit steigenden Konzentrationen des Tensids mit 3 mmol/l, 4,5 mmol/l und 6 mmol/l Caiciumhärte vorbereitet und nach nicht weniger als einer Stunde und nicht mehr als zwei Stunden beobachtet wurde, ob Opaleszenz oder Trübungen aufgetreten sind oder sich sogar Niederschläge gebildet haben. Die Auswertung erfolgt durch Mittelwertbildung der addierten Einzelwerte (5 = klar; 4 = opaleszierend; 3 = trüb; 2 = geringer Niederschlag; 1 = starker Niederschlag;

| DMAP-GA | C12/C14-Glucamid | APG | CAPB | C12-Sarkosinat |
|---|---|---|---|---|
| 5 | 2 | 3 | 5 | 2 |

Die Bestimmung der Beständigkeit gegenüber hartem Wasser zeigt, daß die erfindungsgemäßen Tenside und Tensidgemische über eine sehr gute Calciumverträglichkeit verfügen.

### 3. Netzvermögen (DIN ISO 8022)

Das Tauchnetzvermögen gibt das Maß für die Fähigkeit einer Tensidlösung an, die in einem Gewebe eingeschlossene Luft zu verdrängen, wenn das Gewerbe in die Lösung eingetaucht wird. Das Tauchnetzvermögen eines Tensids wird durch Bestimmung der Netzzeit mit einem Baumwolläppchen in einer Tensidlösung bekannter Konzentration ermittelt.

| Tensid | Netzzeit (Sekunden) |
|---|---|
| C₁₂/C₁₄-DMAP-GA*Milchsäure | 290 |
| C₁₂/C₁₄-DMAP-GA *HCl | 250 |
| C₁₆/C₁₈-DMAP-GA *Milchsäure | > 300 |
| C₁₆/C₁₈-DMAP-GA *HCl | > 300 |
| DMAP-GA | > 300 |
| APG | 120 |
| GA | 100 |
| CAPB | 50 |
| C₁₂-Sarkosinat | 40 |
| Na-LES | 55 |

Die Bestimmung des Netzvermögens zeigt, daß die Tenside der Formel 1 sowie deren Säureaddukte und Mischungen dieser Tenside mit anderen Tensiden über eine vergleichsweise lange Netzzeit verfügen. Dieser Befund ist erfreulich, denn im allgemeinen ist ein gutes Netzvermögen auch mit einer stark entfettenden Wirkung verbunden.

Die Hautfreundlichkeit der Tenside der Formel 1 sowie deren Säureaddukte und Mischungen dieser Tenside mit anderen Tensiden wird durch die Bestimmung des Zeinwertes und des Red-Blood-Cell (RBC)-Wertes bestätigt.

Für DMAP-GA wurde ein Zein-Wert von 39 mg N/100 ml und ein RBC-Wert von 7 % Denaturierung gemessen. Die Werte entsprechen einer sehr guten Hautverträglichkeit.

## Patentansprüche

1. Tensidhaltige Formulierungen enthaltend eine Verbindung der Formel (1) 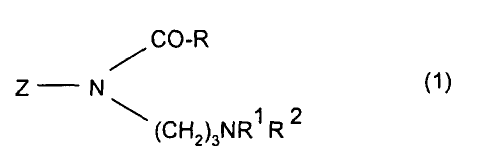 worin R ein aliphatischer Rest mit 8 bis 24 C-Atomen ist, R¹ und R², die gleich oder verschieden sind, Alkyl mit 1 bis 4 C-Atomen oder Hydroxyalkyl mit 2 bis 4 C-Atomen und Z einen linearen Polyhydroxykohlenwasserstoffrest mit mindestens 3 OH-Gruppen, die auch alkoxyliert sein können, bedeuten, sowie zusätlich enthaltend ein oder mehrere anionische, nicht-ionische, kationische und/oder amphotere Tenside.

2. Tensidhaltige Formulierungen nach Anspruch 1, **dadurch gekennzeichnet, daß** R¹ und R² Methyl und Z der Rest eines Zuckeralkohols ist, der sich von einem reduzierenden Mono- oder Disaccharid ableitet.

3. Tensidhaltige Formulierungen nach Anspruch 2, **dadurch gekennzeichnet, daß** das reduzierende Monosaccharid Glucose ist.

4. Tensidhaltige Formulierungen nach einem oder mehreren der Ansprüche 1 bis 3, **dadurch gekennzeichnet, daß** sie Verbindungen der Formel (1) in einer Menge von 0,1 bis 99 Gew.-% enthalten.

5. Tensidhaltige Formulierungen nach Anspruch 4 , **dadurch gekennzeichnet, daß** sie Verbindungen der Formel (1) in einer Menge von 1 bis 50 Gew.-% enthalten.

## Claims

1. A surfactant-containing formulation comprising a compound of the formula (1) 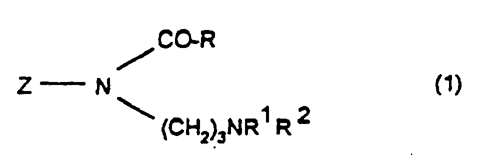 where R is an aliphatic radical having from 8 to 24 carbon atoms, R¹ and R², which are identical or different, are alkyl groups having from 1 to 4 carbon atoms or hydroxyalkyl groups having from 2 to 4 carbon atoms, and Z is a linear polyhydroxyhydrocarbon radical having at least 3 OH groups, which may also be alkoxylated, and also additionally one or more anionic, nonionic, cationic and/or amphoteric surfactants.

2. The surfactant-containing formulation as claimed in claim 1, wherein R¹ and R² are methyl, and Z is the residue of a sugar alcohol derived from a reducing mono- or disaccharide.

3. The surfactant-containing formulation as claimed in claim 2, wherein the reducing monosaccharide is glucose.

4. The surfactant-containing formulation as claimed in one or more of claims 1 to 3, which comprises compounds of the formula (1) in an amount of from 0.1 to 99% by weight.

5. The surfactant-containing formulation as claimed in claim 4, which comprises compounds of the formula (1) in an amount of from 1 to 50% by weight.

## Revendications

1. Formulations tensioactives contenant un composé de formule (1) 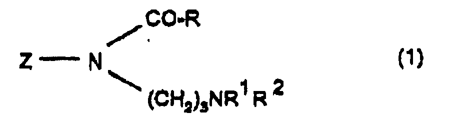 dans laquelle R est un groupe aliphatique ayant de 8 à 24 atomes de carbone, R¹ et R², qui sont identiques ou différents, représentent un groupe alkyle ayant de 1 à 4 atomes de carbone ou hydroxyalkyle ayant de 2 à 4 atomes de carbone et Z représente un groupe polyhydroxyhydro-carboné linéaire ayant au moins 3 groupes OH, qui peuvent aussi être alcoxylés, et contenant également un ou plusieurs agents tensioactifs anioniques, non ioniques, cationiques et/ou amphotères.

2. Formulations tensioactives selon la revendication 1, **caractérisées en ce que** R et R¹ sont un groupe méthyle et Z est le résidu d'un alcool de sucre, qui dérive d'un mono- ou disaccharide réducteur.

3. Formulations tensioactives selon la revendication 2, **caractérisées en ce que** le mono.saccharide réducteur est le glucose.

4. Formulations tensioactives selon une ou plusieurs des revendications 1 à 3, **caractérisées en ce qu'**elles contiennent des composés de formule (1) en une quantité de 0,1 à 99 % en poids.

5. Formulations tensioactives selon la revendication 4, **caractérisées en ce qu'**elles contiennent des composés de formule (1) en une quantité de 1 à 50 % en poids.
